Europäisches Patentamt

European Patent Office

Office européen des brevets

(11) Publication number: **0 082 651**
**B1**

## (12) EUROPEAN PATENT SPECIFICATION

(45) Date of publication of patent specification: **27.05.87**

(21) Application number: **82306618.8**

(22) Date of filing: **10.12.82**

(51) Int. Cl.⁴: **A 61 F 2/20**

(54) **Auto-actuable speech simulator apparatus.**

(30) Priority: **11.12.81 US 329768**

(43) Date of publication of application:
**29.06.83 Bulletin 83/26**

(45) Publication of the grant of the patent:
**27.05.87 Bulletin 87/22**

(84) Designated Contracting States:
**DE FR GB SE**

(56) References cited:
**FR-A- 535 179**
**FR-A- 877 621**
**US-A-1 985 013**
**US-A-4 338 488**

(73) Proprietor: **HANSA MEDICAL PRODUCTS INC.**
**1633 North Capitol Avenue**
**Indianapolis Indiana 46202 (US)**

(72) Inventor: **Blom, Eric Davis**
**8939 Shagbark Road**
**Indianapolis Indiana 46260 (US)**
Inventor: **Singer, Mark Irwin**
**2851 Jamieson**
**Indianapolis Indiana 46260 (US)**
Inventor: **Harkleroad, Barry Arthur**
**7919 Roseway Court**
**Indianapolis Indiana 46226 (US)**

(74) Representative: **Allen, William Guy Fairfax et al**
**J.A. KEMP & CO. 14 South Square Gray's Inn**
**London WC1R 5EU (GB)**

Courier Press, Leamington Spa, England.

## Description

The invention relates to an auto-actuable speech simulator apparatus for tracheotomized patients.

The patient dependent on mechanical ventilation through a tracheostomy is unable to vocalize because of interrupted air flow to the larynx. An inability to communicate may compromise medical care and prove a source of extreme frustration and emotional stress to the patient. It is generally not feasible to disconnect the ventilator, deflate the cuff on the tracheostomy tube and occlude the tube to momentarily allow the patient to exhale around it for brief communication. Moreover, should the patient be a quadriplegic, written and gestural communications are impossible and lip reading or an elimination process of questioning both have their obvious limitations.

Electronic artificial larynges have been developed to provide rudimentary communication for tracheotomized patients. The most common artificial larynx is generally a hand-held, battery-powered instrument which emits a continuous high frequency buzz. The buzzing sound is transmitted through the neck into the mouth where it is modified by mouth and throat movements to produce recognizable speech-like sounds. Difficulty is often encountered, however, in placing the instrument effectively and securely against the skin of the mandibular triangle and coordinating sound production with speech articulation. Importantly, the quadriplegic patient remains dependent on others to anticipate when he wants to speak and to operate the artificial larynx.

Pneumatic artificial larynges have also been developed. The most common of these devices is the Tokyo artificial larynx which has a stoma cover connected to a mouth tube with a vibrator chamber therebetween (see "The artificial larynx: past and present", by Eric Blom, Ph.D, pp. 57—66). A modified version has a finger-controlled breathing port by the vibrator chamber. Both of these devices are hand-held and manually operable.

The obvious limitations inherent in the use of the artificial larynx by a tracheotomized quadriplegic led to the development of an auto-actuable device comprising a headband with an adjustable sliding arm which actuates a tone generator, the output of which is tubed to a patient's mouth. A sliding contact switch mounted on the forehead is utilized to activate the tone generator to produce sound for speech by the simple wrinkling action of the patient's forehead. This device has a major limitation in that the headband must be adjusted snugly enough to stay in place, but not so tight as to restrict circulation. This often necessitates the use of a cotton sweatband or a lightweight cap to increase comfort and to decrease skin irritation and slipping on oily skin or hair. Moreover, the location of the tube within the patient's mouth interferes significantly with the patient's ability to articulate. The opening of the mouth tube frequently fouls with siliva, thus impeding speech production. Additionally, speech produced by this device is electronic sounding and this artificial quality is frequently rejected by potential users.

Alternative devices have also included the Pitt "Speaking Tracheostomy Tube" and the Portex "Trach Talk" which comprise tracheostomy tubes having a narrow gauge conduit incorporated into their convex surface that terminates at a fenestration slightly above the inflatable cuff. An external air flow can be delivered to the larynx through this conduit independent of the oxygen being supplied to the lungs by mechanical ventilation. This is accomplished manually by the patient occluding a "Y" connector attached between the narrow gauge conduit and a source of compressed air. In so doing, sufficient air flow is provided the patient such that an audible whisper might be produced.

In practice, it has been found that the location of a fenestration through which compressed air is delivered to the larynx is situated too close to the tracheostomy, thereby allowing air to escape out and around the tracheostomy tube. This results in decreased air flow for speech and an annoying hissing noise at the neck. Furthermore, patients have frequently reported that air flow reaching the larynx tickles or gags them if, for example, the flow is directed in too narrow a stream. It has been shown that patients on a ventilator frequently have laryngeal and supra-laryngeal tissue changes secondary to traumatic intubation. In many instances these tissue changes interfere with adequate air flow for voice production in patients with whom the "talking trach tubes" are tried. Finally, and most importantly, quadriplegic patients who can achieve audible speech with currently available speaking tracheostomy tubes still remain dependent on others to anticipate when they want to speak and to operate an external air flow.

As an illustration of the prior art, US—A—1985013 discloses an auto-actual speech simulator for a patient comprising a source of pressurized air, a reed, flexible tubing ducting the air past the reed, a tube having a proximal reed end thereof and a distal end thereof for introducing air to the pharynx of the patient, whereby the patient may articulate audibly comprehensible speech. Such a construction has the limitations mentioned above.

According to the present invention, the simulator also includes a regulator governing the pressure of the air, a valve interconnected between said source and said regulator and responsive to actuation by the patient for coupling the vibration in the air induced by operation of the reed into the tube, the valve being operable between a first position wherein no vibrations are coupled from the vibrator to the tube, and a second position wherein vibrations are coupled from the reed to the tube and the tube is in the form of a catheter capable of introducing the vibrating air induced in said catheter by said reed, when the valve is in its second position, to the pharynx of the patient to produce said audibly comprehensible speech.

Such an apparatus is relatively simple to manufacture and can enable a patient readily to give intelligible speech even though the patient is trachiotomized. It is also contemplated that a switch arrangement could be provided and if the trachiotomized patient is also quadriplegic, this switch could be of a type which the patient can actuate other than by hand. Thus the electrical switch which would connect the power supply to an electro-mechanically operable valve could be of a construction including first and second adhesive pads for affixing to an epidermal area of the quadriplegic patient at first spaced apart positions, respectively, and movable towards each other to second relatively lesser spaced apart positions, respectively, by voluntary muscular action of the quadriplegic patient, a field producing device, such as a magnet, secured to said first adhesive pad and a field responsive switch, such as a magnetic reed switch, secured to a second adhesive pad and having an open condition thereof when said adhesive pads are at one of said displaced distances and a closed condition thereof when said adhesive pads are at the other of said displaced distances. Pads could, for example, be placed on the patient's forehead so that the patient can wrinkle his forehead to actuate the switch.

It is important that the catheter should be of the correct length and it is preferably of a length such that it is located, in use, a short distance below the uvula to prevent the patient from gagging and still enable the patient to provide articulate comprehensible speech. The catheter preferably has, adjacent its distal end, a plurality of fenestrations which are of a size and number to prevent pressures in excess of that causing the patient to gag when the distal end is adjacent to the uvula, from building up in the catheter. It is also important that the switching on of the air supply should not cause a rush of air into the patient's pharynx and this can be prevented by mounting the reed in a chamber in a housing to which the tube and catheter are connected, the cross-section of the housing being sufficiently greater than that of the tube and catheter to prevent a surge of air sufficient to cause the patient to gag from initially passing along the catheter.

In order that the invention may more readily be understood, the following description is given, merely by way of example, reference being made to the accompanying drawings, in which:—

Fig. 1 is a diagrammatic representation of a tracheotomized patient illustrating the insertion of a cuffed tracheostomy tube and the tubing, reed housing, and catheter of one embodiment of apparatus according to the invention;

Fig. 2 is a schematic illustration of the air valve and power supply of the apparatus showing the electrical circuitry thereof;

Fig. 3 is a front plan view of the auto-actuable switch of the invention in the normally open position illustrating its affixation to the forehead of a quadriplegic patient;

Fig. 4 is a front plan view of the specific embodiment illustrated in Fig. 3 shows the auto-actuable switch shown in Fig. 3 in the normally closed position;

Fig. 5 is a close-up perspective, partially broken away, and fragmentary view of the tubing, reed housing and catheter shown in Fig. 1; and

Fig. 6 is a sectional view of the tubing, reed housing and catheter shown in Fig. 1 taken substantially along Section Line 6—6 of Fig. 5.

Referring to the drawings, the improved auto-actuable speech simulator 10 of the invention is shown to comprise a compressed air source 12, an air valve 16 and an air pressure regulator 14. Air source 12 furnishes air under pressure through air valve 16 to regulator 14. Regulator 14 conducts air through tubing 18 into reed housing 20 and past reed 98 located in reed chamber 102 to produce vibrating air which is conducted out of the reed chamber 102 through catheter 22 into the pharynx of a tracheotomized patient having a trachostomy tube 8 to provide patient ventilation.

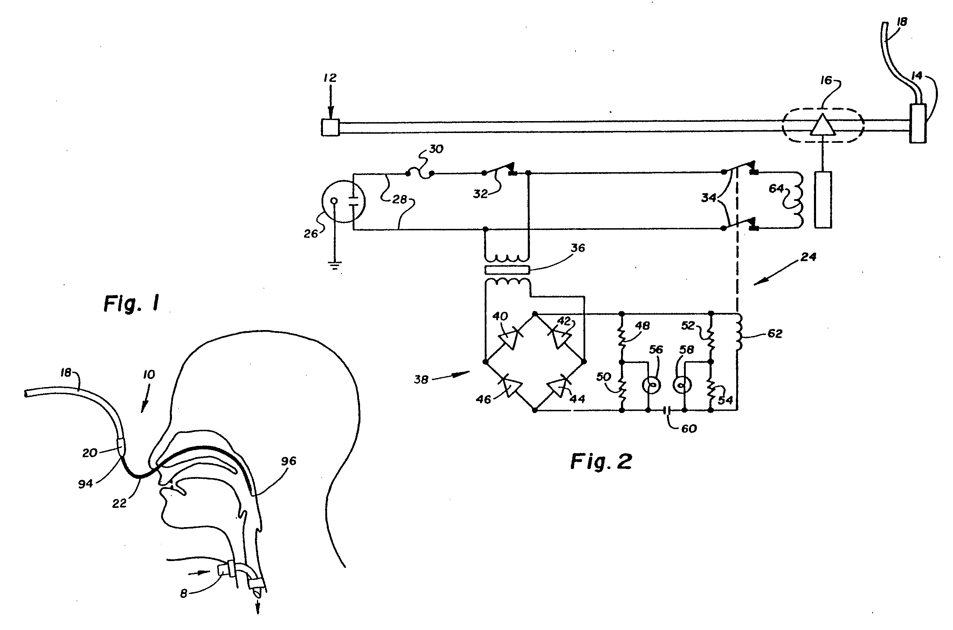

Since vibrating air is delivered to the pharynx of a tracheotomized individual at a relatively low pressure, compressed air source 12 can be one of any number of commercially available compressed air sources. In most specific embodiments, however, the compressed air source 12 is either the readily available compressed air source in patient rooms as found in many hospitals and doctors' offices or portable cylinders of compressed air. In specific embodiments, such air sources furnish air at pressures in the range of from about 2.76 to 6.21 bars. As shown in Fig. 2, air source 12 is connected through valve 16 to regulator 14. Regulator 14, in specific embodiments, may be any commercially available air pressure regulator. When air source 12 is either in the form of available patient room air sources or portable compressed air cylinders, regulator 14 may be one of any of the commercially available air regulators used in conjunction with such compressed air sources for other patient purposes. In many specific embodiments, air source 12 and regulator 14 have associated therewith a humidifier (not shown) to humidify the air prior to its entry into air regulator 14. Moisturized air reduces the drying of the membranes in the pharynx by use of the invention.

Tubing 18 can be any flexible tubing having a wall thickness sufficiently able to withstand the air pressure of air source 12 and to prevent kinking or sidewall collapse during use. Preferred tubing 18 suffers no collapse or kinking when bent into a radius as small as about 12.7 mm. In a specific embodiment, tubing 18 can be of metal reinforced elastomeric materials, i.e. silastic and latex materials. Tubing 18 must be long enough and flexible enough to provide for sufficient patient mobility. In a specific embodiment, tubing 18 is TYGON (Trade Mark) tubing having a 3.2 mm internal diameter such as normally used in hospital rooms in connection with patient room air sources.

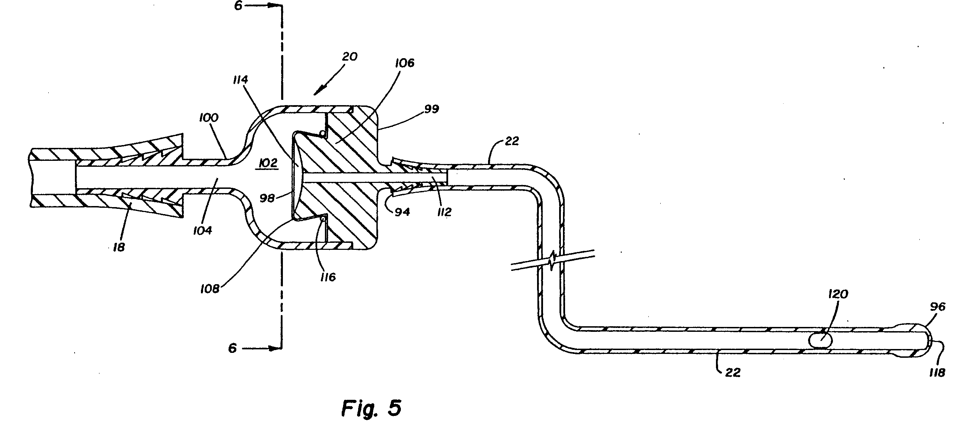

Referring to Fig. 5, reed housing 20, tubing 18 and catheter 22 are shown in detail. Housing 20 has opposite ends 99 and 100 and an interior chamber 102. A pressurized air duct 104 is secured to housing 20 at end 100 and extends into chamber 102. Pressurized air duct 104 is attached to tubing 18 as shown in Fig. 1. Adjacent to end 99 of reed housing 20 is an air outlet 112. A reed support 106 is secured to reed housing 20 at end 100 and extends inwardly of chamber 102. Reed support 106 surrounds pressurized air outlet 112. Reed support 106 has reed supporting surfaces 108 thereon which are spaced from both air pressurized duct 104 and air outlet 112. Reed supporting surfaces 108 face opposite reed chamber end 100 and are spaced from both ends 99 and 100 of reed chamber 102. Air outlet 112 is spaced from end 100 of reed housing 20 and reed supporting surfaces 108. Catheter 22 is attached to air outlet 112.

Reed 98 is supported on reed supporting surfaces 108 and pressurized air flowing into reed chamber 20 through duct 104 flows past reed 98 and out outlet 112 causing reed 98 to vibrate. Supported in this manner, reed 98 and reed support 106 define a reed space 114 therebetween. In the specific embodiment illustrated, reed 98 is of an elastomeric material. Reed 98 is positioned on reed supporting surfaces 108 and is stretched over reed supporting surfaces 108 and held in place by band 116. So held, reed 98 is in tension. The tension of reed 98 can be varied to vary the pitch of reed 98 when vibrating. In all preferred embodiments, the vibration of reed 98 should be adjustable from about 120 cycles per second to about 260 cycles per second. In other embodiments, reed 98 can take the form of a plastic or metal tongue which is secured to the reed supporting surfaces and suspended over outlet 112.

Catheter 22 has a proximal reed end 94 and a distal pharyngeal end 96. Catheter 22 directs the audibly vibrating air from reed 98 into the pharynx of the user. Catheter 22 is inserted in the specific embodiment illustrated through a nostril of the patient into the region of the pharynx such that the patient is able by means of mouth movements to articulate audible speech. Catheter 22 has sidewalls of sufficient strength such that no wall collapse occurs in operation. In a specific embodiment, catheter 22 has sidewalls of sufficient strength such that no wall collapse occurs when the catheter is bent into a radius of about 12.7 mm.

Catheter 22 between proximal end 94 and pharyngeal end 96 is from about 305 mm to about 356 mm long. In addition to the opening 118 in pharyngeal end 96 of catheter 22, catheter 22 has a plurality of fenestrations 120. Fenestrations 120 are of a size and number to prevent pressures in excess of that causing individuals to gag when pharyngeal end 96 is positioned within the pharynx of the user or reed 98 to stop vibrating to build up in catheter 22. In a specific embodiment, catheter 22 has three fenestrations 120 in addition to opening 118 therein formed adjacent to pharyngeal end 96 of catheter 22.

When catheter 22 is correctly positioned within the pharynx of the user, pharyngeal end 96 is positioned to about the level of the uvula. Positioning pharyngeal end 96 significantly below the uvula will cause the user to gag. Positioning pharyngeal end 96 above the uvula either results in no audibly articulate speech or speech which has unwanted noises. Thus, preferably, end 96 should be within about a centimeter of the uvula.

Catheter 22 may be No. 8, 10, 12, or 14 French (Fr) catheters. In a specific embodiment, catheter 22 is of silastic or latex material. No. 8 French catheters are preferred. No. 14 French catheters can be used but are not as comfortable and may give the user a burning sensation when positioned in a nostril and pharynx of the user. Preferably the catheter is positioned in a nostril. While catheter 22 can be also positioned in the mouth, catheter 22 may obstruct during speech articulation; and thus, the positioning of catheter 22 in the nostril is preferred.

In all embodiments of the invention, regulator 14, tubing 18, reed housing 20, and catheter 22 combine to deliver to reed 98 air at a sufficient pressure and flow rate to vibrate the reed from about 256 cycles per second to about 130 cycles per second. Regulator 14, tubing 18, reed chamber 20 and catheter 22 in combination also deliver to the distal pharyngeal end 96 of catheter 22 air at a sufficient air pressure and flow rate to enable the user to articulate audibly comprehensible speech when catheter 22 is positioned in the pharynx as aforementioned, but at a flow rate and air pressure less than that causing the individual to gag or to stop reed 98 from vibrating. This is accomplished by providing pharyngeal end 96 with fenestrations of sufficient size and number and choosing the length and inside diameter of catheter 22 and tubing 18 and the inside dimensions of reed chamber appropriately to result in the proper pressure and flow rate at pharyngeal end 96 at a desired setting of regulator 14.

The flow of air from compressed air source 12 to regulator 14 is controlled by means of air valve 16. Air valve 16 is an electro-mechanical valve operable in response to a power supply actuated by a patient operated electrical switch. In a specific embodiment, the patient operated switch 32 is manually operable single-pole single-throw control switch of the traditional kind of patient operated switch used in most hospitals.

Air valve 16 operates in response to power supply 24. Power is furnished to power supply 24 through grounded plug 26 which is connected to any conventional AC mains supply source. Power from grounded plug 26 is conducted by line cord 28 to power supply 24 and is fused on one lead thereof by means of fuse 30. Single-pole single-throw switch 32 controls the application of power through fuse 30 to stepdown transformer 36 which in the embodiment shown has a secondary output of approximately 24 volts AC.

Secondary output of stepdown transformer 36 is applied to full wave rectifier bridge 38 comprising diodes 40, 42, 44 and 46. Alternately, a battery pack can be utilized in lieu of the AC source and stepdown transformer 36 for portable use.

The rectified output of full wave rectifier bridge 38 is applied to a voltage divider network comprising resistor 48 and resistor 50. The power on indicator 56 is connected in parallel with resistor 50 to indicate the closure of single-pole single-throw switch 32 through the application of power to stepdown transformer 36.

The second voltage divider network comprising series connected resistor 52 and resistor 54 is connected in parallel to the voltage divider network comprising resistor 48 and resistor 50 through switch jack 60. Switch jack 60 allows the application of power to the series connected resistors 52 and 54 by means of the closure of the single pole single throw switch (not shown) connected to switch jack 60. When electrical contact is made across the terminals of switch jack 60, the output of full wave rectifier bridge 38 is applied across resistor 52 and resistor 54 thereby illuminating valve open indicator 58 and energizing relay coil 62. When energized, relay coil 62 causes the closing of relay contacts 34 in the primary circuit of stepdown transformer 36. When a switch connected to switch jack 60 is in the closed position, relay contacts 34 close and power is applied to solenoid coil 64 thereby opening air valve 16 and allowing air to pass from compressed air source 12 to regulator 14. When not energized, solenoid coil 64 allows air valve 16 to close, thereby stopping the flow of air from compressed air source 12 to regulator 14.

Upon the opening and closing of air valve 16, air is supplied to regulator 14 and air moves through tubing 18, reed chamber 102 and catheter 22. When catheter 22 is positioned in the pharynx of an individual as above-described, a sudden surge of air upon the opening of air valve 16 will cause the patient to gag as the flow of air through catheter 22 is at a flow rate or pressure higher than after flow has been maintained for some time. To minimize such sudden surges of air through catheter 22, reed chamber 102 is provided in such a size in relationship to the diameter of tubing 18 and catheter 22 so as to cause air to expand into the reed chamber 102 before passing reed 98 and into the catheter 22 thereby eliminating gagging. Reed chambers 102 larger than necessary to avoid any "sudden surge" which will cause gagging are useful with the invention; however, the delivery of audibly vibrating air to the pharynx will be delayed such that the user must anticipate speech and accordingly close switch 32.

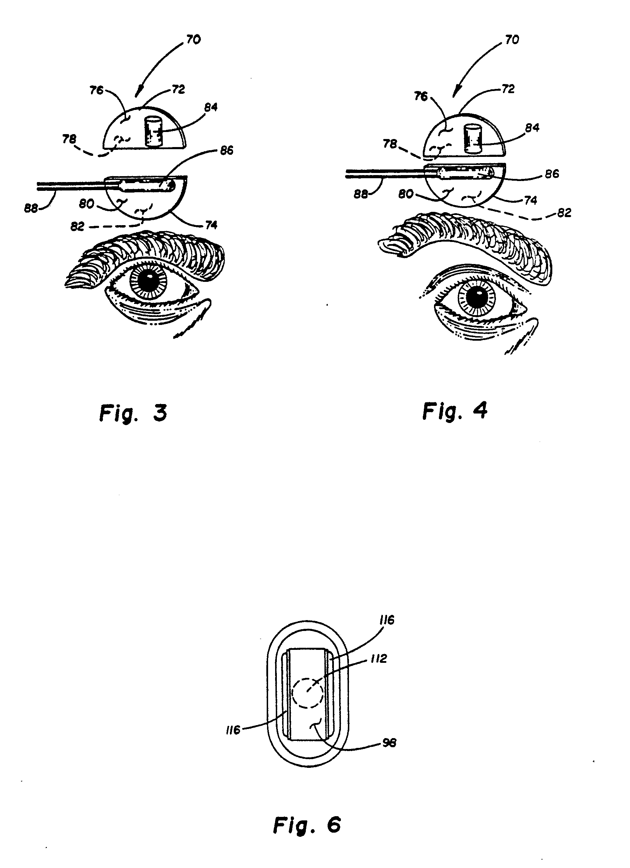

Referring now to Figs. 3 and 4, another embodiment 70 of switch to be connected to switch jack 60 is shown. Switch 70 is designed for actuation without the use of hands. Switch 70 can be used with quadriplegic patients unable to utilize manually operated switches without assistance. Switch 70 comprises an upper pad 72 and a lower pad 74. Upper pad 72 presents a front surface 76 and an adhesively coated back surface 78. Similarly, lower pad 74 presents a front surface 80 and an adhesively coated back surface 82. In use, upper pad 72 and lower pad 74 are affixed to a portion of the users anatomy over which he has voluntary muscle control by adhesively securing back surface 78 and back surface 82 to the patient. In a specific embodiment, since a quadriplegic patient or even the most severe paralyzed individuals are still able to wrinkle the forehead, upper pad 72 may be positioned over a crease line on the patient's forehead with lower pad 74 positioned subjacently. Affixed to either upper pad 72 or lower pad 74 at the front surface 76 or 80, respectively, may be affixed a permanent magnet 84 or other field producing device. Opposite to permanent magnet 84 on either of the counter part lower pad 74 or upper pad 72 on the front surface 80 or 76 respectively is affixed a magentic reed switch 86 or a corresponding field responsive switch. The output of magnetic reed switch 86 is conducted via switch leads 88 to switch jack 60 for operation of the power supply 24.

In use, upper pad 72 and lower pad 74 are positioned so that when the quadriplegic maintains his forehead in an unwrinkled condition, the magnetic reed switch 86 is unaffected by permanent magnet 84 and hence remains in an open position. Subsequently, should the quadriplegic patient wish to activate the auto actuable speech simulator 10 of the invention, the wrinkling of the forehead causes the field permanent magnet 84 to actuate magnetic switch 86, thereby closing the contact between the switch leads 88 and presenting a shorted condition across switch jack 60. This action introduces a vibrating air column to the tracheomized quadriplegic's pharynx and allows the quadriplegic to articulate audibly comprehensible speech.

The invention above described provides an improved speech simulator for tracheotomized patients. The invention provides an improved auto actuable switch, speech simulator which allows oral communication with and readily intelligible speech by a tracheotomized patient, and even a tracheotomized quadriplegic patient. The improved speech simulator can be achieved by a hand switch if possible or by an improved forehead mounted switch by a quadriplegic and other incapacitated patients not able to use a hand switch. The improved speech simulator 10 of the invention provides the introduction of air through a nasal catheter and does not interfere with the patient's speech, or produce unwanted noises or physical sensations during use. Speech is non-electronic and quasi-natural sounding. The speech simulator of the invention provides a device which has few moving parts, may be simply and inexpensively produced and which requires no assistance and little, if any, instruction for use by a tracheotomized patient. The speech simulator of the invention can be used by a quadriplegic patient or even the most severely paralyzed patients and is both convenient and comfortable to use.

## Claims

1. An auto-actuable speech simulator for a patient, comprising a source (12) of pressurized air, a reed (98), flexible tubing (18) ducting the air past the reed (98), a tube (20, 22) having a proximal vibrator end (94) thereof and a distal end (96) thereof for introducing air to the pharynx of a patient, whereby the patient may articulate audibly comprehensible speech, characterised in that the simulator also includes a regulator (14) governing the pressure of the air, a valve (16) interconnected between said source and said regulator and responsive to actuation by the patient for coupling the vibrations in the air induced by operation of the reed (98) into the tube (22), the valve (16) being operable between a first position wherein no vibrations are coupled from the reed (98) to the tube (22) and a second position wherein vibrations are coupled from the reed (98) to the tube (22), and in that said tube (22) is in the form of a catheter capable of introducing the vibrating air induced in said catheter by said reed (98) when the valve (16) is in its second position to the pharynx of the patient, to produce said audibly comprehensible speech.

2. Apparatus according to claim 1, characterised in that said source of pressurized air provides air at a pressure of between 2.76 and 6.21 bars.

3. Apparatus according to claim 1 or 2, characterised in that said tube (18) and catheter (22) both have side walls of sufficient strength such that no side wall collapse occurs when said tubing and catheter are bent into ranges of about 12.7 millimetres.

4. Apparatus according to claim 1, 2 or 3, characterised in that said catheter (22) is from about 305 to about 356 millimetres long and is of a gauge within the range of a number 8 (Fr) to a number 14 (Fr) catheter.

5. Apparatus according to any preceding claim, characterised in that the catheter adjacent to said distal end (96) thereof has a plurality of fenestrations (120) therein, said fenestrations being of a size and number to prevent pressures in excess of that causing individuals to gag when said distal end is adjacent to their uvular from building up in said catheter.

6. Apparatus according to any preceding claim, characterised in that said reed (98) is supported in a chamber (102) in a housing (20) to which the tube (18) and catheter (22) are connected, the cross-section of the housing being sufficiently greater than that of the tube or catheter to prevent a surge of air sufficient to cause the patient to gag from initially passing along the catheter.

7. Apparatus according to any preceding claim, characterised in that said reed (98) is an elastomeric membrane, the tension of which may be adjusted to give the desired pitch.

8. Apparatus according to any preceding claim, characterised in that said valve (16) is electromagnetically operable by connection to a power supply (26) by a patient operated electrical switch (60, 70).

9. Apparatus according to claim 8, characterised in that said electrical switch (60) is a switch (70) for actuation by a quadriplegic patient, said switch comprising first and second adhesive pads (72, 74) for affixing to an epidermal area of said quadriplegic patient at first spaced apart positions, respectively, and movable towards each other to second relatively lesser spaced apart positions, respectively, by voluntary muscular action of said quadriplegic patient, a field producing device (84) secured to said first adhesive pad, a field responsive switch (86) secured to said second adhesive pad and having an open condition thereof when said adhesive pads are at one of said displaced distances and a closed condition thereof when said adhesive pads are at the other of said displaced distances.

10. Apparatus according to claim 9, characterised in that said field producing device (84) comprises a permanent magnet and said field responsive switch 86 comprises a magnetic reed switch.

## Patentansprüche

1. Selbsterregender Sprachsimulator für einen Patienten, die eine Druckluftquelle (12), eine Zunge (98), eine flexible, die Luft an der Zunge (98) vorbei führende Rohrleitung (18) sowie ein Rohr (20, 22) mit einem proximalen Vibratorende (94) und einem distalen Ende (96) zum Einführen von Luft in den Pharynx eines Patienten, aufweist, wodurch der Patient eine verständliche Sprache hörbar von sich geben kann, dadurch gekennzeichnet, daß der Simulator auch einen den Luftdruck steuernden Regler (14) sowie ein zwischen der genannten Druckluftquelle und dem erwähnten Regler angeordnetes Ventil (16) umfaßt, das auf eine Erregung durch den Patienten anspricht, um die durch den Betrieb der Zunge (98) in der Luft induzierten Schwingungen in das Rohr (22) zu übertragen, wobei das Ventil (16) zwischen einer ersten Stellung, in der von der Zunge (98) zu dem Rohr (22) keine Schwingungen übertragen werden, und einer zweiten Stellung, in der von der Zunge (98) zu dem Rohr (22) Schwingungen übertragen werden, betriebsfähig ist, und daß das genannte Rohr (22) die Form eines Katheters aufweist, der in der Lage ist, die schwingende Luft, welche in dem Katheter durch die Zunge (98) induziert worden ist, wenn das Ventil (16) sich in seiner zweiten Stellung befindet, in den Pharynx des Patienten einzuführen, um die genannte hörbar verständliche Sprache hervorzurufen.

2. Vorrichtung nach Anspruch 1, dadurch gekennzeichnet, daß die Druckluftquelle Luft mit einem Druck zwischen 2,76 und 6,21 bar bereitstellt.

3. Vorrichtung nach Anspruch 1 oder 2, dadurch gekennzeichnet, daß sowohl die Rohrleitung (18) als auch der Katheter (22) Seitenwände von ausreichender Stärke aufweist, so daß kein Zusammenfalten der Seitenwände eintritt, wenn die Rohrleitung und der Katheter in Bereichen von etwa 12,7 mm abgebogen werden.

4. Vorrichtung nach Anspruch 1, 2 oder 3, dadurch gekennzeichnet, daß der Katheter (22) eine Länge von etwa 305 bis etwa 356 mm und ein Kaliber eines Katheters im Bereich von Nr. 8 (Fr) bis Nr. 14 (Fr) aufweist.

5. Vorrichtung nach einem der vorstehenden Ansprüche, dadurch gekennzeichnet, daß der dem distalen Ende (96) benachbarte Katheter eine Mehrzahl von Fenstern (120) in einer solchen Anzahl und Größe aufweist, daß in dem Katheter der Aufbau von Überdrucken verhindert wird, die Menschen zum Brechen reizen, wenn das genannte distale Ende ihrer Uvula benachbart ist.

6. Vorrichtung nach einem der vorstehenden Ansprüche, dadurch gekennzeichnet, daß die Zunge (98) in einer Kammer (102) in einem Gehäuse (20), mit dem die Rohrleitung (18) und der Katheter (22) verbunden sind, getragen wird, wobei der Querschnitt des Gehäuses ausreichend größer ist als jener der Rohrleitung oder des Katheters, um zu verhindern, daß ein Luftstoß, der für einen Brechreiz des Patienten ausreicht, zu Beginn entlang des Katheters fortschreitet.

7. Vorrichtung nach einem der vorstehenden Ansprüche, dadurch gekennzeichnet, daß die Zunge (98) eine elastomere Membran ist, deren Spannung einstellbar ist, um die gewünschte Tonlage zu ergeben.

8. Vorrichtung nach einem der vorstehenden Ansprüche, dadurch gekennzeichnet, daß das Ventil (16) durch Anschließen an eine Energiequelle (26) mittels eines von einem Patienten bedienten elektrischen Schalters (60, 70) elektromagnetisch in Betrieb setzbar ist.

9. Vorrichtung nach Anspruch 8, dadurch gekennzeichnet, daß der genannte elektrische Schalter (60) ein Schalter (70) für eine Erregung durch einen an allen vier Gliedmaßen gelähmten Patienten ist, wobei der genannte Schalter ein erstes und ein zweites Klebekissen (72, 74) zur jeweiligen Befestigung an einem epidermalen Bereich des gelähmten Patienten an ersten voneinander beabstandeten Positionen, wobei durch die willkürliche Muskelbewegung des gelähmten Patienten die Klebekissen jeweils gegeneinander zu zweiten, voneinander relativ weniger beabstandeten Positionen bewegbar sind, eine ein Feld erzeugende Vorrichtung (84), die an dem genannten ersten Klebekissen befestigt ist, und ein auf das Feld ansprechender Schalter (86), der an dem genannten zweiten Klebekissen befestigt ist sowie eine offene Lage hat, wenn die Klebekissen sich in der einen der gegeneinander versetzten Entfernungen befinden, und eine geschlossene Lage hat, wenn die Klebekissen sich in der anderen der gegeneinander versetzten Entfernungen befinden, aufweist.

10. Vorrichtung nach Anspruch 9, dadurch gekennzeichnet, daß die das Feld erzeugende Einrichtung (84) einen Permanentmagneten und der auf das Feld ansprechende Schalter (86) einen magnetischen Zungenschalter aufweist.

**Revendications**

1. Simulateur de parole auto-commandé pour un malade, comprenant une source (12) d'air sous pression, une languette (98), un tube flexible (18) conduisant l'air au-delà de la languette (98), un tube (20, 22) ayant une extrémité proximale vibrante (94) et une extrémité distale (96) pour introduire de l'air dans le pharynx d'un malade, le malade pouvant ainsi articuler des paroles compréhensibles et audibles, caractérisé en ce que le simulateur comprend également un régulateur (14) réglant la pression de l'air, une valve (16) interconnectée entre ladite source et ledit régulateur et répondant à la commande par le malade pour transmettre par couplage dans le tube (22) les vibrations produites dans l'air par la mise en oeuvre de la languette (98), la valve (16) fonctionnant entre une première position, dans laquelle aucune vibration n'est transmise par couplage de la languette (98) au tube (22), et une seconde position, dans laquelle des vibrations sont transmises par couplage de la languette (98) au tube (22), et en ce que ledit tube (22) est en forme de cathéter pouvant introduire l'air vibrant produit dans ledit cathéter par ladite languette (98), quand la valve (16) est dans sa seconde position, dans le pharynx du malade, pour produire lesdites paroles compréhensibles et audibles.

2. Appareil selon la revendication 1, caractérisé en ce que ladite source d'air sous pression fournit un air à une pression comprise entre 2,76 et 6,21 bars.

3. Appareil selon la revendication 1 ou 2, caractérisé en ce que lesdits tube (18) et cathéter (22) ont tous deux des parois latérales de résistance suffisante pour qu'il ne se produise aucun aplatissement de paroi latérale quand lesdits tube et cathéter sont pliés en éléments d'environ 12,7 millimètres.

4. Appareil selon la revendication 1, 2 ou 3, caractérisé en ce que ledit cathéter (22) a une longueur d'environ 305 à 356 millimètres et a un calibre compris entre le calibre 8 (Fr) et le calibre 14 (Fr).

5. Appareil selon l'une des revendications précédentes, caractérisé en ce que le cathéter, au voisinage de son extrémité distale (96) susmentionnée, comporte une pluralité d'ouvertures (120), lesdites ouvertures étant d'une dimension et en un nombre tels que l'on empêche la création dans ledit cathéter de pressions excédant celles, qui amènent des individus à bégayer, quand ladite extrémité distale est adjacente à leur luette.

6. Appareil selon l'une des revendications précédentes, caractérisé en ce que ladite languette (98) est, dans une chambre (102), maintenue dans un logement (120) auquel le tube (18) et le cathéter (22) sont reliés, la section transversale du logement étant suffisamment plus grande que celle du tube ou du cathéter pour empêcher le passage initial dans le cathéter d'un à-coup d'air suffisant pour amener le malade à bégayer.

7. Appareil selon l'une des revendications précédentes, caractérisé en ce que ladite languette (98) est une membrane en élastomère, dont la tension peut être réglée pour donner la hauteur de son désirée.

8. Appareil selon l'une des revendications précédentes, caractérisé en ce que ladite valve (16) est commandée électro-magnétiquement par liaison avec une source d'énergie (26) grâce à un interrupteur électrique (60, 70) commandé par le malade.

9. Appareil selon la revendication 8, caractérisé en ce que ledit interrupteur électrique (60) est un interrupteur (70) destiné à être mis en oeuvre par un malade tétraplégique, ledit interrupteur comprenant un premier et un second supports adhésifs (72, 74) destinés à être fixés sur une surface épidermique dudit malade tétraplégique, à des premières positions écartées l'une de l'autre, respectivement, et mobiles l'une vers l'autre pour atteindre des secondes positions moins écartées l'une de l'autre, respectivement, par une action musculaire volontaire dudit malade tétraplégique, un dispositif (84) générateur de champ étant fixé sur ledit premier support adhésif, un interrupteur (86) sensible au champ étant fixé sur ledit second support adhésif et ayant une position ouverte quand lesdits supports adhésifs sont à l'une des distances d'écartement, et une position fermée, quand lesdits supports adhésifs sont à l'autre des distances d'écartement.

10. Appareil selon la revendication 9, caractérisé en ce que ledit dispositif (84) produisant un champ comprend un aimant permanent et ledit interrupteur (86) sensible au champ comprend un interrupteur à languette magnétique.

Fig. 2

Fig. 1

Fig. 3

Fig. 4

Fig. 6

0 082 651

Fig. 5

3